# EUROPEAN PATENT APPLICATION

(11) **EP 3 009 069 A2**
(43) Date of publication of application: **20.04.2016**
(21) Application number: 15190061.0
(22) Date of filing: 16.10.2015
(51) Int. Cl.: A61B 5/00

(54) **WEARABLE SENSOR TO MONITOR BIOSIGNAL AND METHOD TO MONITOR BIOSIGNAL USING WEARABLE DEVICE**

(30) Priority: 16.10.2014 KR 20140139742
(71) Applicant: Samsung Electronics Co., Ltd., Suwon-si, Gyeonggi-do, 443-742 (KR)
(72) Inventor: KIM, Sang Joon, 443-803 Gyeonggi-do (KR); YOON, Kihyun, 443-803 Gyeonggi-do (KR); CHOI, EunHa, 443-803 Gyeonggi-do (KR); YOON, Seungkeun, 443-803 Gyeonggi-do (KR)
(74) Representative: Grünecker Patent- und Rechtsanwälte PartG mbB

(57) **Abstract**

A wearable device to monitor a biosignal includes a wearable sensor configured to monitor the biosignal of an individual and an interactor configured to supply a charging power to the wearable sensor in response to a connection to the wearable sensor.

## Description

### 1. Field

The following description relates to a wearable device and a method to manage a power of the wearable device.

### 2. Description of Related Art

Recently, an interest in wearable devices has increased. A wearable device may refer to a device made to be worn on a body or attached to a garment of a user. The wearable device is made small and lightweight in order for a user to use the device freely in a mobile environment. Accordingly, the wearable device may always stay with the user even though the user is performing a predetermined activity. Further, the user may always use the wearable device without time restrictions. Every time the user uses the wearable device, the wearable device executes a command and provides the user with information.

There are various types of wearable device, for example, a garment type wearable device, an accessory type wearable device, and the like. Also, the wearable device is used in various fields, for example, a fashion field and a medical field. The field in which the wearable device is used is expected to be substantially expanded.

### SUMMARY

This Summary is provided to introduce a selection of concepts in a simplified form that are further described below in the Detailed Description. This Summary is not intended to identify key features or essential features of the claimed subject matter, nor is it intended to be used as an aid in determining the scope of the claimed subject matter.

In one general aspect, there is provided a wearable device to monitor a biosignal of an individual, the wearable device including a wearable sensor configured to monitor the biosignal of the individual, and an interactor configured to supply a charging power to the wearable sensor in response to a connection to the wearable sensor.

The interactor may be further configured to process the biosignal monitored by the wearable sensor and to provide the individual with a result of processing the biosignal, in response to the connection to the wearable sensor.

The wearable sensor may include a processor configured to process the biosignal by consuming an amount of power less than a power threshold.

The wearable sensor may include a communicator configured to transmit data associated with the biosignal by consuming an amount of power less than a power threshold.

The wearable sensor may include a battery configured to supply a power to operate the wearable sensor, and the battery may be configured to receive the charging power from the interactor, in response to a connection to the interactor.

The wearable sensor may be further configured to provide the individual with charging information, in response to a remaining amount of power detected in the battery being less than a remaining threshold.

The interactor may include a processor configured to operate in response to at least one of a processing rate required for a calculation associated with the biosignal being faster than a threshold rate and a memory required for the calculation being greater than a threshold memory.

The interactor may include a communicator configured to transmit data at a data rate higher than a threshold data rate.

The interactor may include a battery configured to supply another power to operate the interactor, and the battery may be further configured to supply the charging power to the wearable sensor in order to charge the wearable sensor, in response to the connection to the wearable sensor.

The interactor may include an interface configured to receive an input from the individual, and a display configured to provide the individual with information associated with an interaction with the individual in response to the input.

The interactor may include at least one of a vibrating motor, a camera, and a microphone to provide an interaction with the individual.

The wearable sensor may include a mounter configured to mount the wearable sensor to a body of the individual.

In another general aspect, there is provided a method to monitor a biosignal by a wearable sensor, the method including monitoring a biosignal of an individual by consuming power of a battery of the wearable sensor, and receiving another power from an interactor, in response to a connection to the interactor.

The method to monitor the biosignal by the wearable sensor may further include transmitting a biosignal monitored by the wearable sensor to the interactor, in response to the connection to the interactor.

The monitoring may include processing the biosignal through a processor consuming an amount of power than less a power threshold.

The method to monitor the biosignal by the wearable sensor may further include transmitting data associated with the biosignal through a communicator consuming an amount of power less than a power threshold.

The method to monitor the biosignal by the wearable sensor may further include providing the individual with charging information, in response to a remaining amount of charge detected in the wearable sensor being less than a remaining threshold,

In still another general aspect, there is provided a method to monitor a biosignal by an interactor, the method including receiving the biosignal from a wearable sensor, in response to a connection to the wearable sensor, processing the received biosignal, and supplying power to the wearable sensor in response to the connection to the wearable sensor.

The processing may include processing the received biosignal through a processor operating in response to at least one of a processing rate required for a calculation associated with the biosignal being faster than a predetermined threshold and a memory required for the calculation being greater than a threshold memory.

The method to monitor a biosignal by an interactor may further include transmitting a result of processing the biosignal through a communicator configured to transmit data at a data rate greater than a threshold data rate.

The wearable device may be further configured to be detachable from the wearable sensor.

Other features and aspects will be apparent from the following detailed description, the drawings, and the claims.

### BRIEF DESCRIPTION OF THE DRAWINGS

FIG. 1 is a block diagram illustrating an example of a configuration of a wearable device to monitor a biosignal.
FIGS. 2 and 3 are diagrams illustrating examples of a structure of a wearable device to monitor a biosignal.
FIGS. 4 and 5 are diagrams illustrating examples of a wearable device to monitor a biosignal.
FIG 6 is a block diagram illustrating an example of a configuration of a wearable device to monitor a biosignal.
FIG. 7 is a block diagram illustrating an example of a configuration of an interactor.
FIG. 8 is a flowchart illustrating an example of a method to monitor a power of a wearable device.
FIG. 9 is a flowchart illustrating an example of a method to monitor a biosignal by a wearable sensor.
FIG 10 is a flowchart illustrating an example of a method to monitor a biosignal by an interactor.

### DETAILED DESCRIPTION

The following detailed description is provided to assist the reader in gaining a comprehensive understanding of the methods, apparatuses, and/or systems described herein. However, various changes, modifications, and equivalents of the systems, apparatuses and/or methods described herein will be apparent to one of ordinary skill in the art. The progression of processing steps and/or operations described is an example; however, the sequence of and/or operations is not limited to that set forth herein and may be changed as is known in the art, with the exception of steps and/or operations necessarily occurring in a certain order. Also, descriptions of functions and constructions that are well known to one of ordinary skill in the art may be omitted for increased clarity and conciseness.

Throughout the drawings and the detailed description, the same reference numerals refer to the same elements. The drawings may not be to scale, and the relative size, proportions, and depiction of elements in the drawings may be exaggerated for clarity, illustration, and convenience.

The features described herein may be embodied in different forms, and are not to be construed as being limited to the examples described herein. Rather, the examples described herein have been provided so that this disclosure will be thorough and complete, and will convey the full scope of the disclosure to one of ordinary skill in the art.

Hereinafter, the examples will be described with reference to the accompanying drawings.

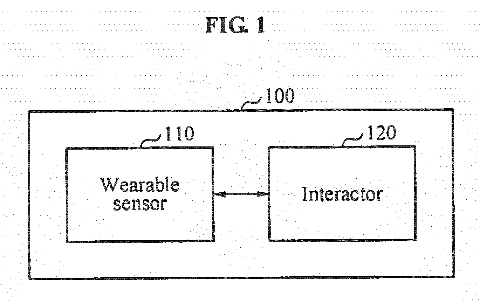

FIG. 1 is a block diagram illustrating a configuration of a wearable device 100 to monitor a biosignal.

In an example, the wearable device 100 refers to an electronic device equipped directly or indirectly to at least a portion of a body. For example, the wearable device directly equipped to at least a portion of a body includes a smart watch and a smart band. The wearable device indirectly equipped to a body, is equipped to, for example, a garment of a body.

In an example, the wearable device 100 is configured to sense a biosignal of a person, and is in the form of various products, such as a smartwatch. Additionally, the wearable device 100 is used in various fields, such as in the health and entertainment fields. However, it is noted that the products and fields are not limited to those described above and thus the wearable device 100 may be in the form of a phone, a tablet, or a personal media player. Similarly, the wearable device 100 may be used in other fields such as the sports field or research fields. Additionally, the wearable device 100 may have issues in a form factor and a use time.

For example, since the wearable device 100 is an electronic device equipped to the human body, a form factor which is smaller and lighter than an existing handheld device is required. Similarly, the wearable device 100 operates in a relatively long-time duration with a single charging cycle, thereby providing a convenience and performing a function to sense consecutive biosignals. Accordingly, the wearable device 100 operates in a relatively longer-term duration, using a form factor smaller than the handheld device.

A method to increase a battery density or decrease a power consumption is employed to secure a long-time duration on a relatively small form factor. Increasing the battery density of a lithium ion (Li-ion) battery is technically difficult since the battery density is at a maximum density due to physical and chemical limitations. Therefore, a method to decrease a power consumption is used. Since each component of the wearable device 100 having functions such as signal processing, communication, and a vibrating motor, has a basic power consumption, decreasing the power consumption has a limitation.

The wearable device 100 to monitor a biosignal includes a wearable sensor 110 to sense consecutive biosignals of a user and an interactor 120 to perform an interaction with the user. The wearable sensor 110 and the interactor 120 are physically separated, thereby increasing the entire time duration. However, this is only an example and the wearable sensor 110 and the interactor 120 may also be combined.

Referring back to the wearable sensor 110 and the interactor 120 being physically separated, for example, the wearable sensor 110 monitors a biosignal of the user for 24 hours based on a purpose, for example, a patient monitoring in medical and health fields. While the wearable sensor 110 always operates by being worn by the user, the interactor 120 operates at a time desired by the user, in response to a request of the user. However, the wearable sensor 110 consumes a lower amount of power than the interactor 120. The wearable sensor 110 processes biosignals at a processing rate of a few hertz (Hz) through hundreds of Hz. However, a processing rate of tens of megahertz (MHz) through hundreds MHz is required for processing visual information on the interaction at the interactor 120.

By way of example, as shown in Table 1, a power consumption of the wearable sensor 110 is configured to be less than 10 milliwatts (mW), and a power consumption of the interactor 120 is configured to be greater than 100 mW. However, this is only an example, and the power consumption of each device may be changed based on design parameters.

**Table 1**

| Module | Use time | Power consumption |
|---|---|---|
| Wearable sensor | Always | Less than 10 mW |
| Interactor | When a user requests | Greater than 100mW |

For example, the wearable device 100 is provided in a form of a band, and the wearable sensor 110 includes a sensor to monitor a biosignal of a user. The interactor 120 provides the user with a user interface (UI) to interact with the user, through a display. Hereinafter, a configuration and an operation of the wearable device 100 to monitor a biosignal will be described in detail.

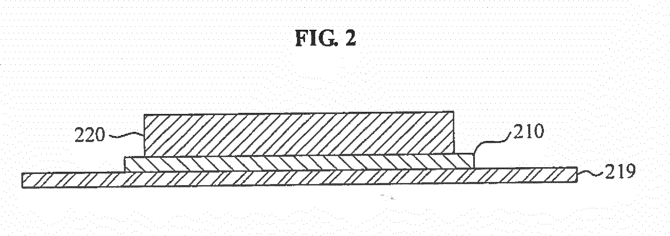

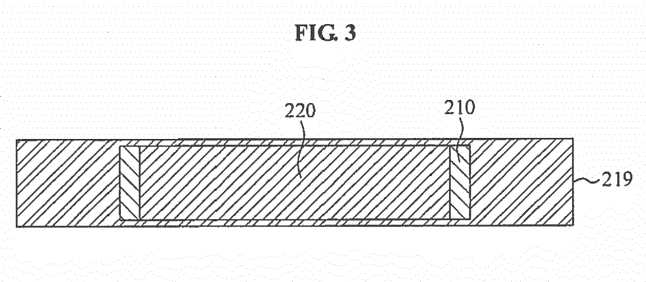

FIGS. 2 and 3 are diagrams illustrating examples of a structure of a wearable device to monitor a biosignal.

The wearable device to monitor the biosignal is provided in a form of which the device is equipped to at least a portion of a body of a user. In an example, the wearable device is provided in a form of a band, a watch, a necklace, a belt, and the like. FIGS. 2 and 3 illustrate examples of a wearable device including a wearable sensor 210 in a form of a band and an interactor 220 which is detachable from the wearable sensor 210.

FIG. 2 illustrates a side view of a wearable device in a form of a band. FIG. 3 illustrates a top view of the wearable device in the form of the band. Referring to FIGS. 2 and 3, a function of monitoring a biosignal of a user and a function of performing an interaction with the user is physically separated based on a module unit. For example, the wearable sensor 210 includes a sensor mounted to a mounter 219 in a form of a band, and an interactor 220 is provided to be detachable from the wearable sensor 210. Based on a need, the user attaches or detaches the interactor 220 to or from the wearable sensor 210.

The mounter 219 mounts the wearable sensor 210 to a body of the user. For example, the mounter 219 mounts the wearable sensor 210 to the body in the form of the band, the watch, the belt, and the like.

According to an example, the wearable sensor 210 in the form of the band senses a biosignal in real time. By separating the wearable sensor 210 and the interactor 220, a battery of the interactor 220 is charged in real time while the wearable sensor 210 is monitoring a biosignal of the user.

As an example, the wearable device is configured by combining the interactor 220 and a single wearable sensor 210 selected by the user from different wearable sensors 210, each wearable sensor 210 including at least one types of sensor. As another example, the wearable device is configured by combining the wearable sensor 210 and a single interactor 220 selected by the user from the different interactors 220 having a plurality of types and functions. As still another example, the wearable device is configured by selecting, by the user, one of the different interactors 220 and one of the wearable sensors 210.

According to an example, the wearable device independently manages each module by separating the wearable sensor 210 and the interactor 220 from each other. When the wearable sensor 210 and the interactor 220 are interconnected, the wearable device uses mutual resources as necessary. Further, a battery of the wearable sensor 210 is charged by a battery of the interactor 220, such that the wearable sensor 210 of the wearable device always operate. However, this is not limited thereto and the battery of interactor 220 may be charged by the battery of the wearable sensor 210.

The wearable sensor 210 and the interactor 220 are connected to each other to be detachable but this is not limited thereto and thus they may be formed as a single unit. For example, the wearable sensor 210 and the interactor 220 are connected in a mechanical structure and a magnetic structure. For example, the mechanical structure includes a connection structure in which the wearable sensor 210 and the interactor 220 are configured to be detachable, as manipulated by the user. The magnetic structure includes a structure in which magnets having detachable magnetism are attached to the wearable sensor 210 and the interactor 220, as controlled by the user, without affecting an electrical operation of the wearable sensor 210 and the interactor 220.

According to an example, when the wearable sensor 210 and the interactor 220 are interconnected, electrical connections are formed between processors and batteries of the wearable sensor 210 and the interactor 220.

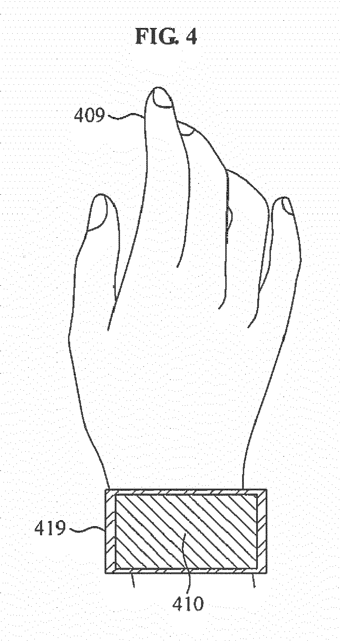

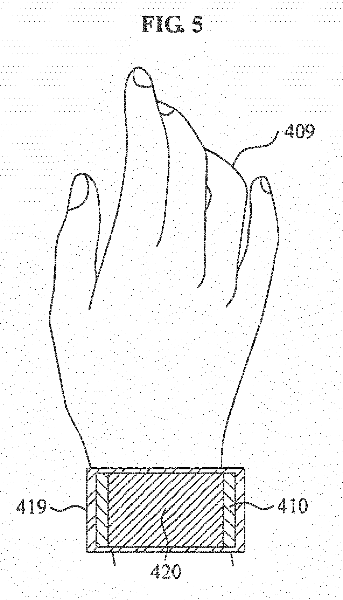

FIGS. 4 and 5 are diagrams illustrating examples of a wearable device to monitor a biosignal.

FIG. 4 illustrates an example in which a user wears a wearable device configured as a wearable sensor 410 around a portion of a body 409. FIG. 5 illustrates an example in which the user wears the wearable device configured as the wearable sensor 410 and an interactor 420 around the portion of the body 409.

For example, in FIG. 4, the wearable device including only the wearable sensor 410 is provided in a form of a band. The wearable sensor 410 is configured to be combined with a mounter 419 in the form of the band. The wearable device is mounted to the portion of the body 409, for example, a wrist of the user, through the mounter 419 in the form of the band.

Referring to FIG. 5, the wearable device of FIG. 4 further includes the interactor 420. For example, the interactor 420 is connected at an upper end of the wearable sensor 410 in the form of the band of FIG. 4. The interactor 420 provides a multimedia service using for example a display and a camera. Also, the interactor 420 provides a service based on the biosignal of the user sensed from the wearable sensor 410.

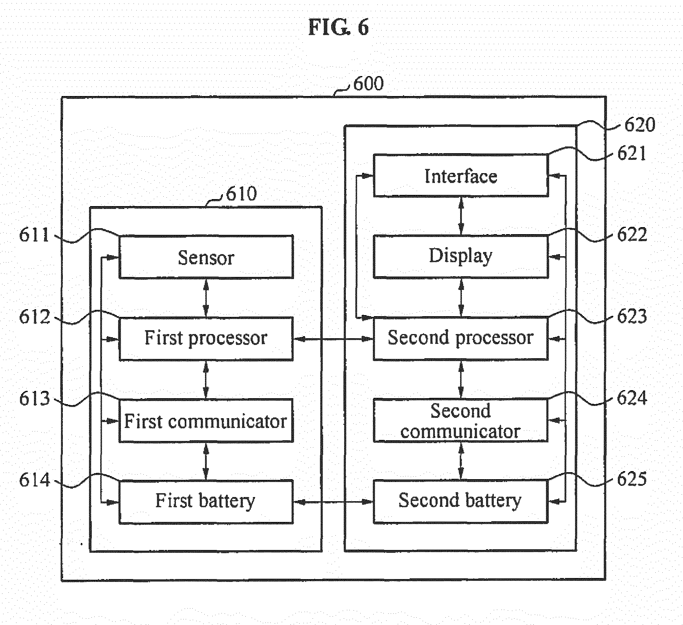

FIG. 6 is a block diagram illustrating an example of a configuration of a wearable device 600 to monitor a biosignal. The wearable device 600 to monitor a biosignal includes a wearable sensor 610 and an interactor 620.

The wearable sensor 610 includes a sensor 611, a first processor 612, a first communicator 613, and a first battery 614. The interactor 620 includes an interface 621, a display 622, a second processor 623, a second communicator 624, and a second battery 625.

The wearable sensor 610 monitors a biosignal of the user. The wearable sensor 610 processes and stores, in real time, the biosignal of the user sensed from the sensor 611. The biosignal of the user includes at least one of bioelectrical signals of the user, for example, an ExG signal, an electrocardiogram (ECG) signal, an electroencephalogram (EEG) signal, an electromyography (EMG), and an electrooculogram (EOG) signal.

The sensor 611 is an electrical element or an electronic element configured to sense the biosignal of the user. For example, the sensor 611 is configured to sense at least one of a bioelectrical signal, an optical biosignal, a skin temperature signal, a bio-impedance signal, and a pressure signal.

The first processor 612 processes the biosignal sensed from the sensor 611. For example, the first processor 612 filters noise from the sensed biosignal and performs preprocessing of the sensed biosignal, such as amplifying the biosignal.

According to an example, the first processor 612 performs the preprocessing while operating in a low power. As an example, the first processor 612 is configured to process the biosignal by consuming a lower amount of power than a predetermined first power threshold. Here, the first power threshold is a predetermined value to limit an operation power of the first processor 612 and is changed based on a design.

The first communicator 613 transmits data associated with the biosignal to an external device (not shown). For example, the first communicator 613 directly transmits the biosignal sensed from the sensor 611 to the external device, or transmits a result of preprocessing biosignal by the first processor 612 to the external device. The external device may be an electronic device independent from the wearable device 600. However, this is only an example and thus the external device may be the interactor 620.

According to an example, the first communicator 613 operates in a low power and transmits data associated with the biosignal to the external device. For example, the first communicator 613 is configured to transmit data associated with the biosignal by consuming a lower amount of power than a predetermined second power threshold. Here, the second power threshold is a predetermined value to limit an operation power of the first communicator 613 and may be changed based on design parameters.

For example, the first communicator 613 transmits data associated with the biosignal to the external device based on an ultra low power communication method or a Bluetooth low energy (BLE) method. However, a communication method of the first communicator 613 is not limited to the ultra low power communication method or the BLE method. For example, any communication method that consumes a lower amount of power than the predetermined second power threshold, may be applied.

For example, as described above with reference to Table 1, the wearable sensor 610 is configured so that a total amount of power consumed by each of the sensor 611, the first processor 612, and the first communicator 613 is less than 10 mW.

The first battery 614 provides a power to operate the wearable device 600. The first battery 614 is charged by a power received from an outside. For example, the first battery 614 receives a charging power from the interactor 620, in response to a connection to the interactor 620 but is not limited thereto. That is, the first battery 614 may be charged, for example, by a movement of the user. The first battery 614 includes a low volume battery. For example, the first battery 614 is charged with an amount of power at which the wearable sensor 610 is operable for at least a predetermined amount of time, for example, 24 hours.

According to an example, the wearable sensor 610 provides the user with charging information, when the battery 614 is determined to be charged with an insufficient amount of power. For example, the wearable sensor 610 provides the user with charging information, in response to a remaining amount of power detected in the first battery 614 being less than a predetermined remaining threshold. The wearable sensor 610 transmits the charging information to an external device through the first communicator 613, or provides the user with the charging information through an additional output device, for example, a speaker.

The remaining amount of the first battery 614 refers to a current amount of power the first battery 614 is charged with, and the predetermined remaining threshold refers to an amount of power insufficient to operate the wearable sensor 610. The charging information includes information associated with a remaining amount, an operable time, and the like of the first battery 614.

The interactor 620 performs an interaction with the user. For example, the interactor 620 provides an interaction based on a biosignal sensed from the wearable sensor 610. According to an example, the interactor 620 is detachable from the wearable sensor 610, and provides power to the wearable sensor 610 in response to a connection to the wearable sensor 610.

The interaction refers to a predetermined operation provided in response to a control, an action, a state, and an emotion of the user. For example, the interactor 620 verifies a sleep state, for example, a sleep cycle, of the user based on the biosignal sensed from the wearable sensor 610, and then performs the interaction of playing an alarm sound and the like, when the sleep state of the user is determined to be suitable for waking up. However, the interaction is not limited to the foregoing. The interaction suitable for the control, the action, the state, the emotion, and the like of the user may be determined based on a design.

The interface 621 is configured to receive an input from the user, and the display 622 is configured to provide the user with, for example, display on a screen, information associated with the interaction with the user in response to the input. Referring to FIG. 6, the interface 621 and the display 622 are illustrated as separate configurations. However, this is only an example and the interface 621 and the display 622 may be configured as an integrated module, for example, a touch screen.

The second processor 623 receives and processes the biosignal sensed from the wearable sensor 610. For example, in response to the connection to the wearable sensor 610 through the second processor 623, the interactor 620 processes the biosignal being monitored by the wearable sensor 610 and provides the user with a result of processing the biosignal.

According to an example, the second processor 623 operates in a high performance and performs a complex calculation using a relatively large power consumption associated with the biosignal, instead of the wearable sensor 610. For example, the second processor 623 is configured to operate at a processing rate, that is, a processing speed faster than a predetermined threshold rate. Here, the processing rate refers to a rate at which the second processor 623 processes data, and the predetermined threshold rate is a value of limiting a minimum rate performance of the second processor 623 and is changed based on a design. Also, the second processor 623 is configured to process the calculation requiring a memory larger than a predetermined threshold memory. Here, the threshold memory is larger than a memory, and smaller than or equal to a size of a memory of the second processor 623.

The second processor 623 is configured to operate in response to at least one of a case in which a processing rate required for a calculation associated with the biosignal is faster than a predetermined threshold rate and a case in which a memory required for the calculation is greater than a threshold memory.

The second communicator 624 transmits data associated with the biosignal to the external device. For example, the second communicator 624 transmits data associated with the biosignal received from the wearable sensor 610 and the result of processing the biosignal through the first processor 612 to the external device, or transmits the result of processing the biosignal through the second processor 623 to the external device. Also, the second communicator 624 transits data processed by the interactor 620 to the external device based on a control of the second processor 623 or receives required information from the external device.

According to an example, the second communicator 624 operates in a high performance and transmits data associated with a biosignal to the external device or receives information from the external device. For example, the second communicator 624 is configured to transmit data at a data rate higher than a predetermined threshold data rate. Here, the threshold data rate is a value limiting a minimum communication rate of the second communicator 624 and is changed based on design parameters.

For example, the second communicator 624 receives and transmits data using a wireless method, such as wireless fidelity (Wi-Fi), and Bluetooth. However, a communication method of the second communicator 624 is not limited to the aforementioned methods. A high performance communication method, for example, all communication methods to receive and transmit data at a data rate higher than the predetermined threshold data rate are applicable to the communication method of the second communicator 624.

The second battery 625 provides a power to operate the interactor 620. The second battery 625 is charged by the power received from the outside but is not limited thereto. That is, the second battery 625 may be charged from power received from the wearable sensor 610 or even from movement of the user wearing the wearable device 100. The second battery 625 provides power to the first battery 614 of the wearable sensor 610 in order to charge the wearable sensor 610, in response to the connection to the wearable sensor 610. Here, the second battery 625 includes a large volume battery. For example, the second battery 625 charges with an amount of power at which the interactor 620 is operable for at least a predetermined amount of time, for example, 12 hours.

According to an example, each of the wearable sensor 610 and the interactor 620 are independently managed, but is not limited thereto. That is, both, the wearable sensor 610 and the interactor 620 can be commonly managed. For example, referring to FIG. 6, the wearable sensor 610 includes the first communicator 613 and the first battery 614, and the interactor 620 includes the second communicator 624 and the second battery 625, to communicate with the external device.

When the wearable sensor 610 and the interactor 620 are interconnected, a data interface between the first processor 612 and the second processor 623 included in each module of the wearable sensor 610 and the interactor 620 may be formed. The wearable sensor 610 processes the biosignal using a resource, for example, the high performance second processor 623 of the interactor 620 having a high specification in response to the connection to the interactor 620. For example, when the wearable sensor 610 senses the ECG signal as the biosignal, the first processor 612 of the wearable sensor 610 performs preprocessing, for example, filtering and the like. The second processor 623 of the interactor 620 performs the following complex calculation. For example, the complex calculation includes calculations having a high complexity, such as a feature extraction, a feature classification, and the like.

Also, with respect to communication with the external device, the wearable device 600 uses the high performance second communicator 624 of the interactor 620, instead of the low power first communicator 613 embedded within the wearable sensor 610 and having a limited bandwidth but is not limited thereto. That is, the wearable device 600 may use the low power first communicator 613 or a combination of both communicators.

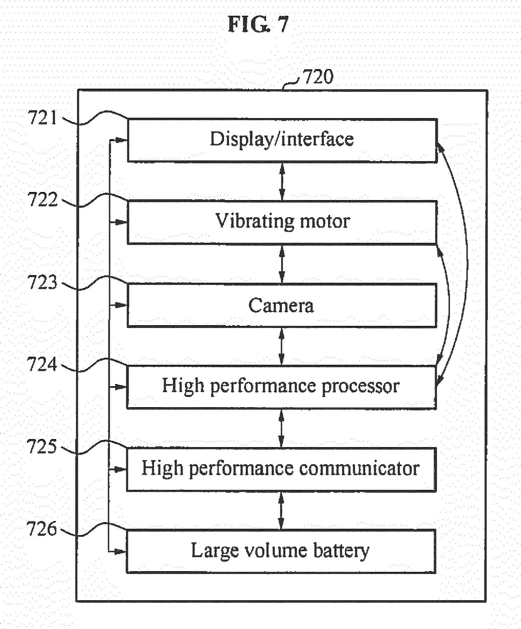

FIG. 7 is a block diagram illustrating an example of a configuration of an interactor 720.

An interactor 720 includes a display/interface 721, a vibrating motor 722, a camera 723, a high performance processor 724, a high performance communicator 725, and a large volume battery 726. The high performance processor 724, the high performance communicator 725, and the large volume battery 726 are configured to be similar to the second processor 623 of FIG 6, the second communicator 624, and the second battery 625, respectively. Additionally, although FIG. 7 illustrates the display/interface 721, the vibrating motor 722, the camera 723, the high performance processor 724, the high performance communicator 725, and the large volume battery 726 as included in the interactor 720, one or more of these elements may be embodied as independent hardware. A wearable sensor detachable from the interactor 720 is configured to be similar to the wearable sensor 610 of FIG. 6.

The display/interface 721 integrally provides functions of the display 621 and the display 622 of FIG. 6. For example, the display/interface 721 includes a touch screen.

The vibrating motor 722 and the camera 723 provide a user with an interaction corresponding to a user action and the like. For example, the vibrating motor 722 provides the user with a vibration based on a control of the high performance processor 724 with respect to the user action. The camera 723 provides the user with a photographing function, an image recording function, and the like, in response to the control of the high performance processor 724 with respect to the user action. However, a module to provide an interaction is not limited to the vibrating motor 722 and the camera 723 and may include various devices, such as a microphone (not shown) to record a voice of the user and a speaker (not shown) to provide the user with a sound, and the like.

Since the wearable sensor needs to measure a biosignal at all times, the wearable sensor should be mounted to the user for a maximally long-time duration. According to an example, the large volume battery 726 of the interactor 720 charges a low volume battery of the wearable sensor, for example, the first battery 614 of FIG. 6, of the wearable sensor, thereby extending the time duration of the wearable sensor.

For example, when the wearable sensor is used alone, the low volume battery included in the wearable sensor is used. Prior to the low volume battery being discharged, the wearable sensor is connected to the interactor 720. Subsequently, through the large volume battery 726 of the interactor 720, the entire wearable device including the wearable sensor and the interactor 720 operate, and the low volume battery of the wearable sensor may be simultaneously charged. When the interactor 720 is removed from the wearable sensor in response to a request of the user or a discharging of the large volume battery 726, the wearable sensor continuously senses the biosignal using the charged low volume battery.

As described above, when the interactor 720 is connected to the wearable sensor prior to the low volume battery of the wearable sensor completely being discharged, the wearable device always monitors the biosignal.

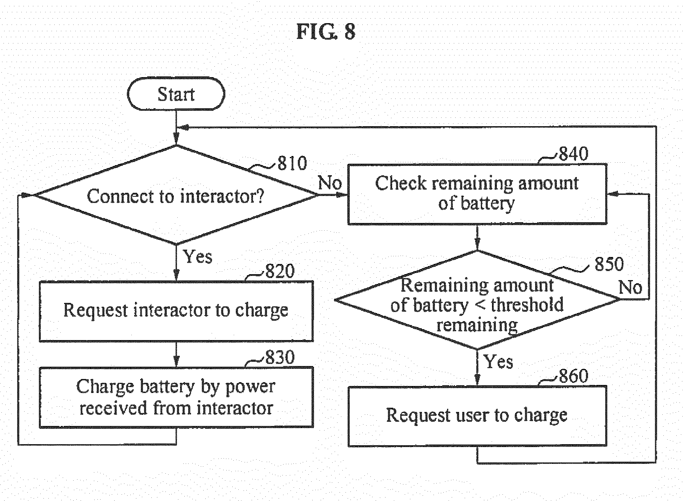

FIG. 8 is a flowchart illustrating an example of a method to manage a power of a wearable device.

In operation 810, a first processor of a wearable sensor determines whether the wearable sensor is connected to an interactor. For example, the first processor senses whether a data interface is formed with a second processor of the interactor. However, this is only an example and thus, the first processor or the second processor detects whether the wearable sensor and the interactor are interconnected based on an electrical method, an electronic method, and a mechanical method.

For example, the electrical method may include a method to sense a predetermined electrical signal generated in response to a connection between the wearable sensor and the interactor. The electronic method may include a method to sense whether a data interface is formed between the processors in response to the aforementioned connection. The mechanical method may include a method to switch ON or OFF a mechanical switch and the like in response to the aforementioned connection.

In operation 820, when the wearable sensor is connected to the interactor, the first processor of the wearable sensor sends a request to the interactor to be charged. For example, the first processor transmits a signal requesting the second processor of the interactor to provide a charging power.

In operation 830, the first battery of the wearable sensor is charged by a power received from the interactor. The second processor may control the second battery to provide the power to the first battery, in response to a charging request by the first processor. The second battery transmits the power to the first battery through an electrical connection formed in response to the connection between the wearable sensor and the interactor. The first battery is charged using the received power. For example, the first battery is charged until the first battery is fully charged.

In operation 840, when the wearable sensor is not connected to the interactor, the first processor of the wearable sensor checks the remaining amount of the battery. The wearable sensor disconnected from the interactor operates only by an internal battery, for example, the first battery, and continuously checks the remaining amount of the first battery. For example, the wearable sensor checks the remaining amount of the first battery every predetermined cycle.

In operation 850, the first processor of the wearable sensor determines whether the remaining amount of the battery is less than a remaining threshold. Here, the remaining threshold may be the predetermined remaining threshold of FIG 6. In an example, when the remaining amount of the battery is less than the predetermined remaining threshold, the first processor determines that the power of the first battery is insufficient to operate the wearable sensor. In another example, when the remaining amount of the battery is more than or equal to the remaining threshold, the first processor continuously checks the remaining amount of the battery, by returning to operation 840.

In operation 860, the first processor of the wearable sensor sends a request to a user requesting the user to charge the wearable sensor. For example, the first processor provides the user with charging information. The first processor directly provides the charging information, for example, generating a warning sound indicating that charging is needed, to the user, or provides the charging information to an external device through the first communicator. When the charging information is provided to the external device through the first communicator, the external device requests the user to charge the wearable sensor based on the charging information.

According to an example, a scenario in which the user wears the wearable sensor and the interactor during the daytime and wears only the wearable sensor during the nighttime is shown in Table 2.

**Table 2**

| Time zone | Daytime | Nighttime |
|---|---|---|
| | 7 a.m. (waking up)∼ | 7 p.m. (returning home)∼ |
| | 7 p.m. (returning home) | 7 a.m. (waking up) |
| A configuration of a wearable device | A wearable sensor and an interactor | The wearable sensor |
| A battery to be used | Using a battery of the interactor | Using a battery of the wearable sensor |
| An operation of the interactor | Charging the wearable sensor, and supplying a power to the wearable device | Charging a battery of the interactor |
| An operation of wearable sensor | Charging the battery of the wearable sensor and monitoring a biosignal | Monitoring the biosignal |

As shown in Table 2, for example, a user wears a wearable device provided in a form of a band and including a wearable sensor and an interactor during the daytime, and the wearable device provides nearly all services. A power of the entire wearable device is supplied by a second battery of the interactor.

Subsequently, the user removes the interactor from the wearable sensor during nighttime after returning home and before sleeping, and charges the interactor through an extra charging device. Since then the wearable sensor operates by the first battery autonomously embedded in the wearable sensor, and monitors a biosignal. During the nighttime, the wearable sensor monitors a biosignal of the user and stores information associated with the monitored biosignal.

When the daytime comes again, the user connects the wearable sensor and the interactor completely charged overnight, and charges the first battery of the wearable sensor which has consumed power overnight, through the second battery. Here, the power to operate the entire wearable device is supplied by the second battery of the interactor.

As described above, the wearable device according to an example provides the user with a service appropriate for each circumstance for 24 hours.

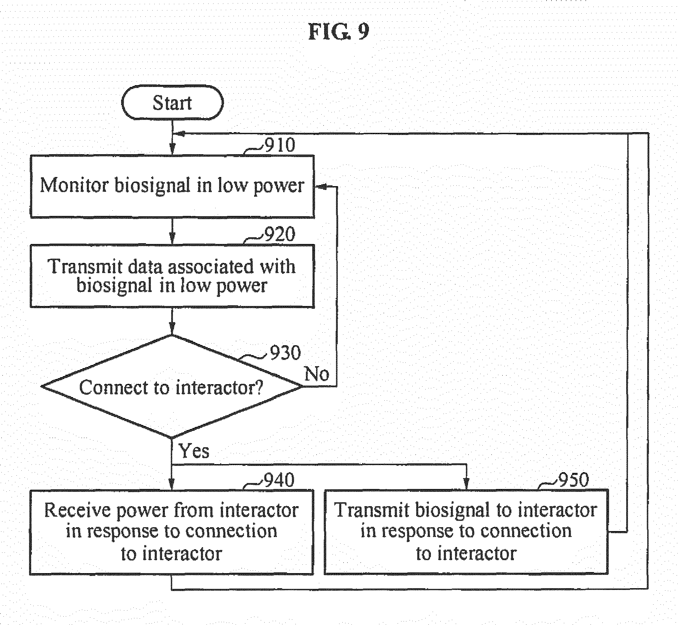

FIG 9 is a flowchart illustrating an example of a method to monitor a biosignal by a wearable sensor.

In operation 910, the wearable sensor monitors the biosignal in a low power. For example, the wearable sensor senses the biosignal through a sensor, and processes the sensed biosignal through a first processor consuming a lower amount of power than a first power threshold.

In operation 920, the wearable sensor transmits data associated with the biosignal in a low power. For example, the wearable sensor transmits the data associated with the biosignal to an external device, through a first communicator consuming a lower amount of power than a second power threshold.

In operation 930, the wearable sensor determines whether the wearable sensor is connected to the interactor. Here, when the wearable senor is determined to be disconnected from the interactor, the wearable sensor continuously monitors the biosignal, returning to operation 910.

In operation 940, the wearable sensor receives power from the interactor in response to a connection to the interactor. For example, when the wearable sensor and the interactor are connected, the wearable sensor receives the power from a second battery of the interactor and charges a first battery of the wearable sensor.

In operation 950, the wearable sensor transmits the biosignal to the interactor in response to the connection to the interactor. For example, when the wearable sensor is connected to the interactor, the wearable sensor transmits the biosignal, the data associated with the biosignal, and the like to the interactor.

However, the description of FIG 9 is not limited to an example of an operation of the wearable sensor. Operations of FIG. 9 may be combined with operations of FIGS. 1 through 8 based on design parameters.

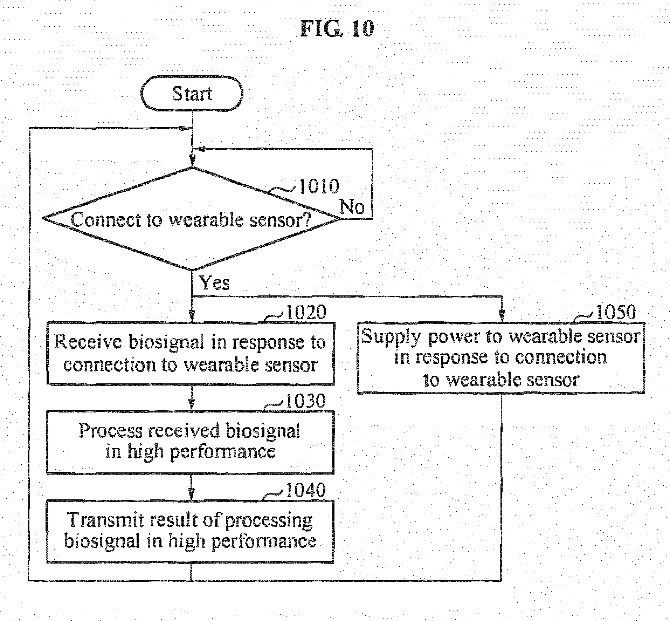

FIG. 10 is a flowchart illustrating an example of a method to monitor a biosignal by an interactor.

In operation 1010, the interactor determines whether the interactor is connected to a wearable sensor. Prior to connecting to the wearable sensor, a second battery of the interactor is charged by an external power source.

In operation 1020, the interactor receives a biosignal in response to a connection to the wearable sensor. For example, the interactor receives the biosignal through a data interface formed between a first processor of the wearable sensor and a second processor of the interactor. Also, the interactor receives the biosignal transmitted from a first communicator of the wearable sensor through a second communicator of the interactor. However, a method and route of biosignal reception of the interactor are not limited to foregoing.

In operation 1030, the interactor processes the received biosignal in a high performance. For example, the interactor processes the received biosignal through the second processor operating at a processing rate faster than a predetermined threshold rate.

In operation 1040, the interactor transmits a result of processing the biosignal in a high performance. For example, the interactor transmits the result of processing the biosignal through the second communicator configured to transmit data at a data rate higher than a predetermined threshold data rate.

In operation 1050, the interactor supplies a power to the wearable sensor in response to the connection to the wearable sensor. For example, the interactor supplies a power of the second battery to the first battery, through an electrical connection formed between the first battery of the wearable sensor and the second battery of the interactor. However, it is noted that the connection is not limited to an electrical connection and other types of connections may be used to supply the power, such as a magnetic connection. The first battery is charged by the power of the second battery.

A processing device is implemented using one or more general-purpose or special-purpose computers, such as, for example, a processor, a controller and an arithmetic logic unit, a digital signal processor, a microcomputer, a field-programmable array, a programmable logic unit, a microprocessor, or any other device capable of running software or executing instructions. The processing device may run an operating system (OS), and may run one or more software applications that operate under the OS. The processing device may access, store, manipulate, process, and create data when running the software or executing the instructions. For simplicity, the singular term "processing device" may be used in the description, but one of ordinary skill in the art will appreciate that a processing device may include multiple processing elements and multiple types of processing elements. For example, a processing device may include one or more processors, or one or more processors and one or more controllers. In addition, different processing configurations are possible, such as parallel processors or multi-core processors.

Software or instructions for controlling a processing device to implement a software component may include a computer program, a piece of code, an instruction, or some combination thereof, for independently or collectively instructing or configuring the processing device to perform one or more desired operations. The software or instructions may include machine code that may be directly executed by the processing device, such as machine code produced by a compiler, and/or higher-level code that may be executed by the processing device using an interpreter. The software or instructions and any associated data, data files, and data structures may be embodied permanently or temporarily in any type of machine, component, physical or virtual equipment, computer storage medium or device, or a propagated signal wave capable of providing instructions or data to or being interpreted by the processing device. The software or instructions and any associated data, data files, and data structures also may be distributed over network-coupled computer systems so that the software or instructions and any associated data, data files, and data structures are stored and executed in a distributed fashion.

The methods according to the above-described embodiments may be recorded, stored, or fixed in one or more non-transitory computer-readable media that includes program instructions to be implemented by a computer to cause a processor to execute or perform the program instructions. The media may also include, alone or in combination with the program instructions, data files, data structures, and the like. The program instructions recorded on the media may be those specially designed and constructed, or they may be of the kind well-known and available to those having skill in the computer software arts. Examples of non-transitory computer-readable media include magnetic media such as hard disks, floppy disks, and magnetic tape; optical media such as CD ROM disks and DVDs; magneto-optical media such as optical discs; and hardware devices that are specially configured to store and perform program instructions, such as read-only memory (ROM), random access memory (RAM), flash memory, and the like. Examples of program instructions include both machine code, such as produced by a compiler, and files containing higher level code that may be executed by the computer using an interpreter. The described hardware devices may be configured to act as one or more software modules in order to perform the operations and methods described above, or vice versa.

A number of examples have been described above. Nevertheless, it should be understood that various modifications may be made. For example, suitable results may be achieved if the described techniques are performed in a different order and/or if components in a described system, architecture, device, or circuit are combined in a different manner and/or replaced or supplemented by other components or their equivalents. Accordingly, other implementations are within the scope of the following claims.

While this disclosure includes specific examples, it will be apparent to one of ordinary skill in the art that various changes in form and details may be made in these examples without departing from the scope of the claims. The examples described herein are to be considered in a descriptive sense only, and not for purposes of limitation. Descriptions of features or aspects in each example are to be considered as being applicable to similar features or aspects in other examples. Suitable results may be achieved if the described techniques are performed in a different order, and/or if components in a described system, architecture, device, or circuit are combined in a different manner and/or replaced or supplemented by other components or their equivalents. Therefore, the scope of the disclosure is defined not by the detailed description, but by the claims.

## Claims

1. A wearable device to monitor a biosignal of an individual, the wearable device comprising:
a wearable sensor configured to monitor the biosignal of the individual; and
an interactor configured to supply a charging power to the wearable sensor in response to a connection to the wearable sensor.

2. The wearable device of claim 1, wherein the interactor is further configured to process the biosignal monitored by the wearable sensor and to provide the individual with a result of processing the biosignal, in response to the connection to the wearable sensor, and/or wherein the interactor is further configured to be detachable from the wearable sensor.

3. The wearable device of claim 1 or 2, wherein the wearable sensor comprises a processor configured to process the biosignal by consuming an amount of power less than a power threshold, and/or
wherein the wearable sensor further comprises a communicator configured to transmit data associated with the biosignal by consuming an amount of power less than a power threshold.

4. The wearable device of one of claims 1 to 3, wherein the wearable sensor comprises a battery configured to supply a power to operate the wearable sensor, and
the battery is configured to receive the charging power from the interactor, in response to a connection to the interactor, and/or
wherein the wearable sensor is further configured to provide the individual with charging information, in response to a remaining amount of power detected in the battery being less than a remaining threshold.

5. The wearable device of one of claims 1 to 4, wherein the interactor comprises a processor configured to operate in response to at least one of a processing rate required for a calculation associated with the biosignal being faster than a threshold rate and a memory required for the calculation being greater than a threshold memory, and/or wherein the interactor further comprises a communicator configured to transmit data at a data rate higher than a threshold data rate.

6. The wearable device of one of claims 1 to 5, wherein the interactor comprises a battery configured to supply another power to operate the interactor, and
the battery is further configured to supply the charging power to the wearable sensor in order to charge the wearable sensor, in response to the connection to the wearable sensor.

7. The wearable device of one of claims 1 to 6, wherein the interactor comprises:
an interface configured to receive an input from the individual; and
a display configured to provide the individual with information associated with an interaction with the individual in response to the input, and/or wherein the interactor further comprises at least one of a vibrating motor, a camera, and a microphone to provide an interaction with the individual.

8. The wearable device of one of claims 1 to 7, wherein the wearable sensor comprises a mounter configured to mount the wearable sensor to a body of the individual.

9. A method to monitor a biosignal by a wearable sensor, the method comprising:
monitoring a biosignal of an individual by consuming power of a battery of the wearable sensor; and
receiving another power from an interactor, in response to a connection to the interactor.

10. The method of claim 9, further comprising:
transmitting a biosignal monitored by the wearable sensor to the interactor, in response to the connection to the interactor.

11. The method of claim 9 or 10, wherein the monitoring comprises processing the biosignal through a processor consuming an amount of power less than a power threshold.

12. The method of one of claims 9 to 11, further comprising:
transmitting data associated with the biosignal through a communicator consuming an amount of power less than a power threshold, and/or providing the individual with charging information, in response to a remaining amount of charge detected in the wearable sensor being less than a remaining threshold.

13. A method to monitor a biosignal by an interactor, the method comprising:
receiving the biosignal from a wearable sensor, in response to a connection to the wearable sensor;
processing the received biosignal; and
supplying power to the wearable sensor in response to the connection to the wearable sensor.

14. The method of claim 13, wherein the processing comprises processing the received biosignal through a processor operating in response to at least one of a processing rate required for a calculation associated with the biosignal being faster than a predetermined threshold and a memory required for the calculation being greater than a threshold memory.

15. The method of claim 13 or 14, further comprising:
transmitting a result of processing the biosignal through a communicator configured to transmit data at a data rate greater than a threshold data rate.
